(19)

Europäisches
Patentamt
European
Patent Office
Office européen
des brevets

(11)     **EP 2 950 119 B1**

(12)                **EUROPEAN PATENT SPECIFICATION**

(45) Date of publication and mention
of the grant of the patent:
**18.05.2016  Bulletin 2016/20**

(51) Int Cl.:
***G01T 1/29*** (2006.01)

(21) Application number: **14169907.4**

(22) Date of filing: **26.05.2014**

(54) **System and method for verifying a particle beam**

System und Verfahren zur Überprüfung eines Partikelstrahls

Système et procédé permettant de vérifier un faisceau de particules

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**02.12.2015  Bulletin 2015/49**

(73) Proprietor: **ION BEAM APPLICATIONS S.A.**
**1348 Louvain-la-Neuve (BE)**

(72) Inventors:
• **Janssens, Guillaume**
**1348 Ottignies-Louvain-la-Neuve (BE)**
• **Prieels, Damien**
**1490 Court-Saint-Etienne (BE)**
• **Smeets, Julien**
**5004 Bouge (BE)**
• **Vander Stappen, François**
**1050 Bruxelles (BE)**

(74) Representative: **Decrock, Patrick**
**IBA**
**Chemin du Cyclotron 3**
**1348 Louvain-la-Neuve (BE)**

(56) References cited:
**EP-A1- 2 140 913        WO-A1-2012/104416
WO-A1-2015/040225    WO-A2-2012/152938
US-A1- 2011 057 110    US-A1- 2012 025 076**

• **MIN CHUL HEE ET AL: "Development of
array-type prompt gamma measurement system
forrange verification in proton therapy",
MEDICAL PHYSICS, AIP, MELVILLE, NY, US, vol.
39, no. 4, 1 April 2012 (2012-04-01), pages
2100-2107, XP012160962, ISSN: 0094-2405, DOI:
10.1118/1.3694098 [retrieved on 2012-03-26]**

EP 2 950 119 B1

**Description**

**Field of the invention**

The invention relates to a particle beam system for delivering energetic particles or bunches of particles to a target. More specifically, the invention is related to a system comprising detectors for detecting prompt gammas. The invention also relates to a method for verifying a penetration depth of an energetic particle beam in a target by detecting prompt gammas produced when the particle beam penetrates the target.

**Description of prior art**

A device for measuring prompt gammas is known from for example reference US8481951B2 or reference WO2012/104416A1 where prompt gammas are measured with a camera that is using one or more collimators. As there exists a known correlation between the emission of prompt gammas and the penetration depth of the particle beam in a target, the penetration depth or the variation of a penetration depth can be deduced by observing prompt gamma data.

A collimator camera with a slit-shaped opening to measure prompt gammas is also described in WO2012/152938A2 wherein a calculation module is provided to determine the position of the slit relative to the target prior to the measurement of the prompt gammas. A dose profile in the direction of the beam line is derived from the detected prompt gammas and from that profile the beam range is determined.

However, those prompt gamma cameras are heavy due to the collimators and the detection efficiency is generally poor. In addition, as discussed by Gueth et al in Phys. Med. Biol. 58 (2013) pages 4563 to 4577, the prompt gamma profiles measured with those collimator type of cameras are sensitive to for example the target position or the target anatomy. In this reference document it is proposed to define a library of calculated references profiles to interpret a measured profile.

In US 2012/025076A1, a laser-driven accelerator system is discussed where it is assumed that particle pulses with high timing resolution could be provided. A detection setup is described using a first detection device for detection a proton beam and a second detection device for detection prompt gammas. A time of flight signal (TOF) can be determined using the first detector as a start signal and the second detector as a stop signal. It is suggested to correlate this TOF signal with the penetration depth of the particle beam. However, no measurement of such a signal is disclosed and no information on the effects of target structure or target inhomogeneities on the shape of the signal is provided.

Therefore there is room to develop a method and device that is more robust and less complex than the known prior art prompt gamma cameras.

**Summary of the invention**

The invention is related to a particle beam system and a method as disclosed in the appended claims.

The invention is firstly related to a particle beam system for delivering energetic particles or bunches of energetic particles to a target. This particle beam system comprises

- a particle beam source for delivering energetic particles or bunches of energetic particles;
- beam directing means for directing the energetic particles or bunches of energetic particles in a beam direction pointing to said target;
- a timing controller configured for providing a timing reference signal representing a time structure for delivering said energetic particles or bunches of energetic particles;
- a first gamma detector configured for detecting prompt gammas emitted from said target.
- a second gamma detector configured for detecting prompt gammas emitted from said target and located with respect to said first detector such that the first and the second detector are detecting prompt gammas emitted from different angles with respect to said beam direction;
- a data acquisition system for measuring prompt gammas in synchrony with the said timing reference signal so as to obtain a timing profile indicating a number of prompt gammas measured as a function of a time elapsed since the delivery of a particle or a bunch of particles, said first and second detector are coupled to said data acquisition system so as to acquire a first timing profile from the first detector and to acquire a second timing profile from the second detector;

The particle beam system is characterised in that it comprises:

- a data analyser comprising an algorithm configured for

o determining from said first timing profile a first time width ΔT1;
o determining from said second timing profile a second time width ΔT2;
o determining a photon travel shift ADP defined as a distance travelled by a photon in the time interval equal to the difference between the first and the second time width:

$$\Delta DP \; = \; (\Delta T2 - \Delta T1) \, {}^{\star} c$$

whereby c is equal to the speed of light;
o determining a particle beam penetration depth by correlating said photon travel shift ΔDP with the difference in detector location between the first and second detector.

In this way, by measuring prompt gammas in synchrony with a reference timing signal representing the time structure of the delivery of the particles or bunches, the signal to noise ratio is strongly improved and moreover there is no need for complex prompt gamma cameras using collimators as currently known in the art. Moreover, by determining a particle penetration depth in a target based on a travel time of a photon, the time the particle travels in the target does not play a role and hence the penetration depth is obtained in a robust way independent from the detailed target structure. For example, a same variation of penetration depth can be caused by different reasons, for example by an overall density variation of the target, or by a cavity in the proximal part of the target, or a cavity in the distal part of the target, which all result in different travel times of the particle beam in the target.

According to a further embodiment, the data analyser comprises computing means configured for calculating a distance R in the target by solving the equation

$$\Delta DP \; = \; \sqrt{(R * R + d1 * d1 - 2 * R * d1 * \cos(\alpha)} \; - $$
$$\sqrt{(R * R + d2 * d2 - 2 * R * d2 * \cos(\beta)} \; - (d1 - d2)$$

whereby

ΔDP is the said photon travel shift;
d1 is the distance from the first detector to the entrance point, the entrance point being defined as the point where the energetic particles or bunches of energetic particles enter the target;
d2 is the distance from the second detector to the entrance point;
a is the angle between the beam direction and the direction going from the entrance point towards the first detector;
β is the angle between the beam direction and the direction going from the entrance point towards the second detector.

According to an embodiment, the data acquisition system is configured to measure the timing profiles with a resolution equal or smaller than ten nanoseconds.
According to an embodiment, the second gamma detector is located with respect to the first detector such that the first and the second detector are detecting prompt gammas emitted at angles with respect to the beam direction that differ by at least 20°. In this way, the detection sensitivity to measure a penetration depth of a particle beam is improved.
According to an embodiment, the first gamma detector is located for measuring prompt gammas emitted at an angle equal or larger than 100° with respect to the beam direction and the second gamma detector is located for measuring prompt gammas emitted at an angle equal or smaller than 80° with respect to the beam direction.
The invention is equally related to a method for verifying a penetration depth of an energetic particle beam in a target by detecting prompt gammas produced when the energetic particle beam penetrates the target, the method comprising the steps of

- providing a first gamma detector configured for detecting prompt gammas;
- providing a second gamma detector configured for detecting prompt gammas;
- locating said first and second detector in a different location with respect to the target such that the first and the second detector are configured for detecting prompt gammas emitted at different angles with respect a direction of the energetic particle beam;
- providing a timing reference signal representing a time structure of the energetic particle beam;
- measuring prompt gammas emitted from the target with said first detector in synchrony with said timing reference signal so as to obtain a first timing profile;

- measuring prompt gammas emitted from the target with said second detector in synchrony with said timing reference signal so as to obtain a second timing profile;
- determining from said first timing profile a first time width ΔT1;
- determining from said second timing profile a second time width ΔT2;
- determining a photon travel shift ΔDP defined as a distance travelled by a photon in the time interval equal to the difference between the first and the second time width:

$$\Delta DP = (\Delta T2 - \Delta T1) * c$$

whereby c is equal to the speed of light;

- determining said penetration depth by correlating said photon travel shift ΔDP with the difference in detector location between the first and second detector.

According to a further method of the invention, the step of determining the penetration depth comprises a step of calculating a distance R in the target by solving the equation

$$\Delta DP = \sqrt{(R * R + d1 * d1 - 2 * R * d1 * \cos(\alpha)} -$$

$$\sqrt{(R * R + d2 * d2 - 2 * R * d2 * \cos(\beta)} - (d1 - d2)$$

whereby

ΔDP is the said photon travel shift
d1 is the distance from the first detector to the entrance point, the entrance point being defined as the point where the energetic particle beam enters the target;
d2 is the distance from the second detector to the entrance point;
α is the angle between the beam direction and the direction going from the entrance point towards the first detector;
β is the angle between the beam direction and the direction going from the entrance point towards the second detector.

According to an embodiment of the method of the invention, the second gamma detector is located with respect to the first gamma detector such that the first and the second detector are detecting prompt gammas emitted at angles with respect to the beam direction that differ by at least 20°.

According to an embodiment of the method of the invention, the first gamma detector is located for measuring prompt gammas emitted at an angle equal or larger than 100° with respect to the beam direction and said second gamma detector is located for measuring prompt gammas emitted at an angle equal or smaller than 80° with respect to the beam direction.

## Brief description of the figures

These and further aspects of the invention will be explained in greater detail by way of example and with reference to the accompanying drawings in which:

Fig.1    schematically illustrates the invention;
Fig.2    shows an example prompt gamma emission and prompt gamma detection profiles;
Fig.3    shows an example prompt gamma emission and prompt gamma detection profiles whereby a cavity is present in the target;
Fig. 4    shows differences in the width ΔT2-ΔT1 of timing profiles according to the invention;
Fig. 5    shows differences in sensitivity between a single detector and a two-detector setup;
Fig. 6    shows differences in width ΔT2-ΔT1 for various setup configurations.

The figures are not drawn to scale. Generally, identical components are denoted by the same reference numerals in the figures.

## Detailed description of the invention

According to a first aspect of the invention a particle beam system for delivering a particle beam to a target is provided. The particle beam system is configured for measuring a penetration depth of a particle beam in a target by measuring prompt gammas emitted when the beam penetrates the target.
According a second aspect of the invention, a method for verifying a penetration depth of an energetic particle beam in a target by detecting prompt gammas is provided.

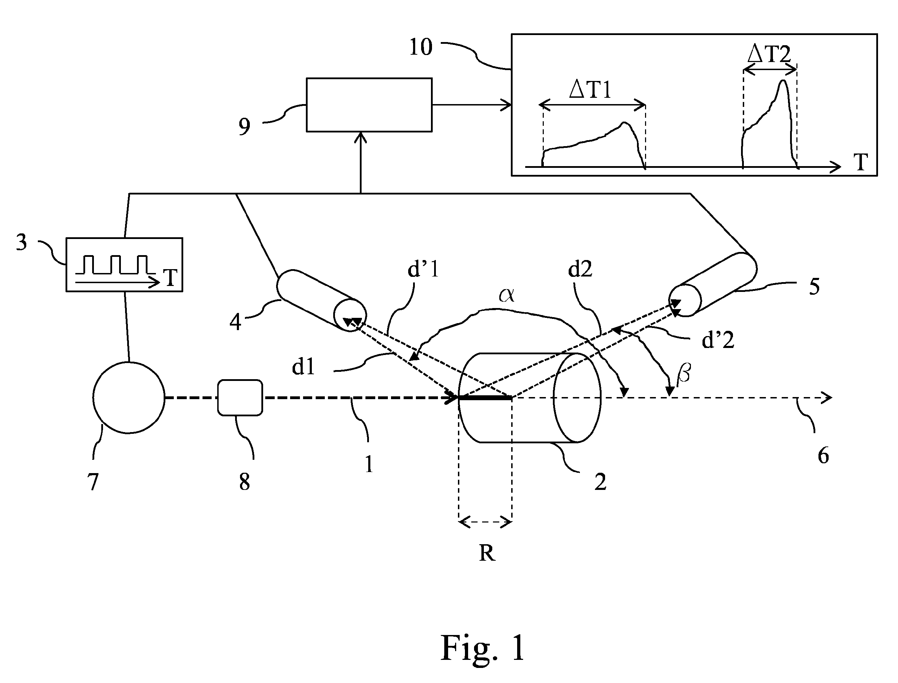

A particle beam system according to the invention is schematically illustrated in Fig. 1. The particle beam system according to the invention is comprising a particle beam source 7 for delivering energetic particles or bunches of energetic particles. Those energetic particles or bunches of energetic particles are forming an energetic particle beam 1. The particle beam source can for example comprise a cyclotron, a synchrotron, a synchrocyclotron, a linear accelerator or any type of accelerator accelerating particles for providing an energetic particle beam.

Under particle beam is understood a charged hadron beam, for example a proton beam or a carbon ion beam. The energy of the beam can vary, depending on the type of particle and on the penetration depth to be reached in the target. The particle beam system and method according to the invention can be used for particle therapy in order to determine the penetration depth of a particle or a bunch of particles in a patient.

In general, the particle beam source comprises a particle accelerator for delivering protons or ions having an energy larger than 40 MeV per mass unit (mu). For example, for the use of proton beams in particle therapy, the energy of the proton beam can typically vary between 50 MeV and 250 MeV. For heavier beams, such as carbon ion beams, the energy of the beam per mass unit (mu) can typically vary between 80 MeV/mu and 450 MeV/mu.

The particle beam source has a time structure for delivering the particles or bunches of particles. For example when the accelerator is a cyclotron, the particle beam source is delivering particles or bunches of particles having a time structure defined by or correlated with the RF frequency of the RF system of the accelerator. For example, a proton cyclotron can have an RF operating frequency in the range of 100 MHz.

The particle beam system further comprises a timing controller 3 configured for providing a timing reference signal representing the time structure for delivering the energetic particles or bunches of energetic particles. The timing reference signal can be a signal derived from the RF signal of the RF system of the accelerator.

In another embodiment, an additional beam buncher can be used to adapt the time structure for delivering the bunches of particles. The beam buncher can for example optimize the length of the bunch. Preferably, the width of the bunch of the particles is smaller than 200 ps (picoseconds) and more preferably the width of the bunch is equal or smaller than 100 ps (picoseconds).

Alternatively, a detector can be used to intercept the particles of the beam and to generate a timing start signal each time a particle is intercepted. Such a detector is sometimes called a hodoscope. By intercepting particle per particle and associating a start signal to each particle, a timing reference signal representing the time structure for delivering the particles is obtained on a particle per particle base.

The particle beam system according to the invention further comprises beam direction means 8 for directing the particles or bunches of particles in a beam direction 6 pointing the target 2. Such a directing means can for example comprise a beam line to transport the beam and a scanning magnet for delivering the particles or bunches of particles to the target. The delivery means can also comprise a gantry rotatable around the target so as to direct the beam from different rotational angles to the target.

The particle beam system further comprises a first gamma detector 4 configured for detecting prompt gammas from the target and a second gamma detector 5 configured for detecting prompt gammas from the target. The first detector and the second detector are located at a different location with respect to the beam direction 6. The first and second detector are located such that the first and the second detector are oriented for detecting prompt gammas emitted at different angles with respect to the beam direction, as illustrated in Fig. 1.

The particle beam system further comprises a data acquisition system 9 for measuring prompt gammas in synchrony with the timing reference signal so as to obtain a timing profile indicating a number of prompt gammas measured as a function of the time elapsed since the delivery of a particle or a bunch of particles. The first and second detector are coupled to the data acquisition system so as to acquire a first timing profile from the first detector and to acquire a second timing profile from the second detector.

The particle beam system according to the invention comprises a data analyser 10 comprising an algorithm configured for determining from the first timing profile a first time width $\Delta T1$ and determining from the second timing profile a second time width $\Delta T2$. The algorithm is further configured for determining a photon travel shift $\Delta DP$ defined as the distance travelled by a photon in the time interval equal to the difference between the first and the second time width, i.e. $\Delta DP = (\Delta T2 - \Delta T1) * c$, whereby c is equal to the speed of light. The algorithm of the data analyser 10 is further configured for determining a penetration depth in the target by correlating the photon travel shift $\Delta DP$ with the difference in detector location between the first and second detector.

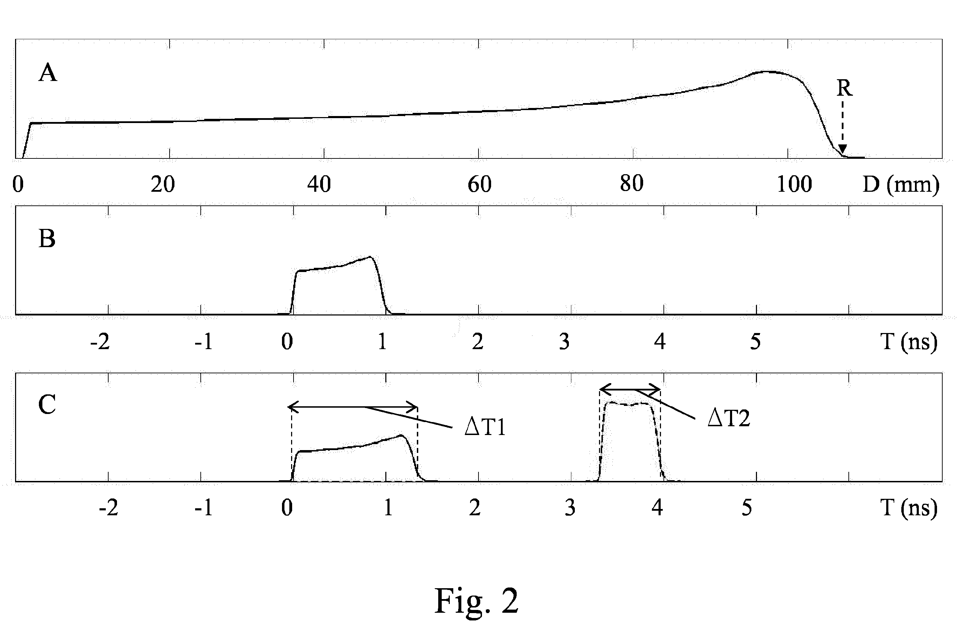

Under correlating the photon travel shift $\Delta DP$ with the difference in detector location between the first and second detector is understood that the photon travel shift can be expressed as depending on one hand on the distances the prompt gammas have to travel from the target to the first and second detector and on the other hand depending on a distance R in the target corresponding to maximum depth in the target from where prompt gammas have been emitted. It is well known in the art that this distance R is closely related to the penetration depth of the particle beam in the target as shown for example in Fig.2(a) of Gueth et al, Phys. Med. Biol. 58 (2013) pages 4563 to 4577.

The invention is further illustrated in Fig. 2 which corresponds to a simulation of a 120 MeV proton beam in a tissue target. In this example, the first detector was located at angle of 180° with respect to the beam direction and the second detector was located at an angle of 0° with respect to the beam direction. The first detector was put on the front side of the target (distance zero) and the second detector was put at a distance of 100 cm from the target entrance. The top panel A shows the relative number of prompt gamma emissions as a function of the depth D in the target, the depth is expressed in mm. The depth R where no further prompt gammas are emitted is indicated on the figure. The middle panel B shows the relative number of prompt gammas emitted as function of time after entering the target. In this example, as can be seen on the middle panel B, prompt gammas are emitted during a time period of about 1 ns (one nanosecond). This time period correspond to the time the particle beam travels in the target until it is stopped when it has lost all its energy in the target.

The lower panel C of Fig. 2 shows the timing profiles as detected with the first detector and the second detector by measuring the prompt gammas in synchrony with the timing reference signal representing the time structure of the particle beam delivered to the target. As can be seen, the widths of the two timing profiles $\Delta T2$ and $\Delta T1$ are different. In this example, the two timing profiles in the lower panel C are clearly separated in time due to the large distance of the second detector from the target. In other examples, the second detector can also be put much closer to the target in which case the two timing profiles may overlap.

The correlation between the photon travel shift $\Delta DP$ and the difference in detector location between the first and second detector can be understood from the illustrative geometry shown in Fig. 1. The first time width $\Delta T1$, measured by the first detector is the difference in time a prompt gamma photon has to travel when either emitted at the entrance of target or emitted at the maximum depth R in the target where prompt gammas are emitted. Following the geometry comprising the distances d1,d2,d'1,d'2 and the two angles $\alpha$ and $\beta$ illustrated in Fig. 1, the time widths can be expressed as follows:

$$\Delta T1 = d1/c - (Tbeam + d'1/c) \qquad (1)$$

$$\Delta T2 = d2/c - (Tbeam + d'2/c) \qquad (2)$$

Whereby Tbeam is the time the particle beam travels in the target until it stops when having lost all its energy. This is the penetration depth of the particle beam in the target. As discussed before, the penetration depth of the particle beam is very closely related to the maximum depth position R of emission of prompt gammas.

In the equations (1) and (2), c is the speed of light, d1 is the distance from the first detector to the entrance point, and d2 is the distance from the second detector to the entrance point. The entrance point is defined as the point where the particle beam enters the target. As further illustrated in Fig.1, d'1 is the distance between the maximum depth R where prompt gammas are emitted and the first detector while d'2 s the distance between the maximum depth R and the second detector.

By taking the difference between $\Delta T2$ and $\Delta T1$, one obtains that

$$\Delta T2 - \Delta T1 = (d2/c) - Tbeam - (d'2/c) - (d1/)c + Tbeam + (d'1/)c$$

Or

$$\Delta T2 - \Delta T1 = (d2/c) - (d'2/c) - (d1/)c + (d'1/c) \qquad (3)$$

In other words, this time difference is independent from the particle beam travel time Tbeam in the target.

As discussed above, a photon travel shift $\Delta DP$ can be defined as

$$\Delta DP = (\Delta T2 - \Delta T1) * c$$

Or

$$\triangle DP = d2-d'2-d1+d'1 \qquad (4)$$

Following standard trigonometric rules one finds that

$$\triangle DP = \sqrt{(R * R + d1 * d1 - 2 * R * d1 * \cos(\alpha)} -$$

$$\sqrt{(R * R + d2 * d2 - 2 * R * d2 * \cos(\beta)} - (d1-d2) \qquad (5)$$

Whereby, as discussed above, R is a distance in the target corresponding to maximum depth in the target from where prompt gammas have been emitted. This is the unknown distance in the target that can be determined with the device and method of the invention. To find R from the above equation number (5), the inventors have used a numerical method to find R.

Preferably, the data analyser (10) according to the invention comprises computing means configured for calculating this distance R in the target by solving the equation number (5) deduced above.

The width $\triangle T1$ and the width $\triangle T2$ can be determined in various ways. For example, the timing profiles can be fitted with a curve and the begin and end point of the profile can be deduced. Alternatively, the width can be defined by defining the begin point and the end point of the profile as being a point having a given value with respect to the average peak value, for example defining the begin and end point as being the 5% level points.

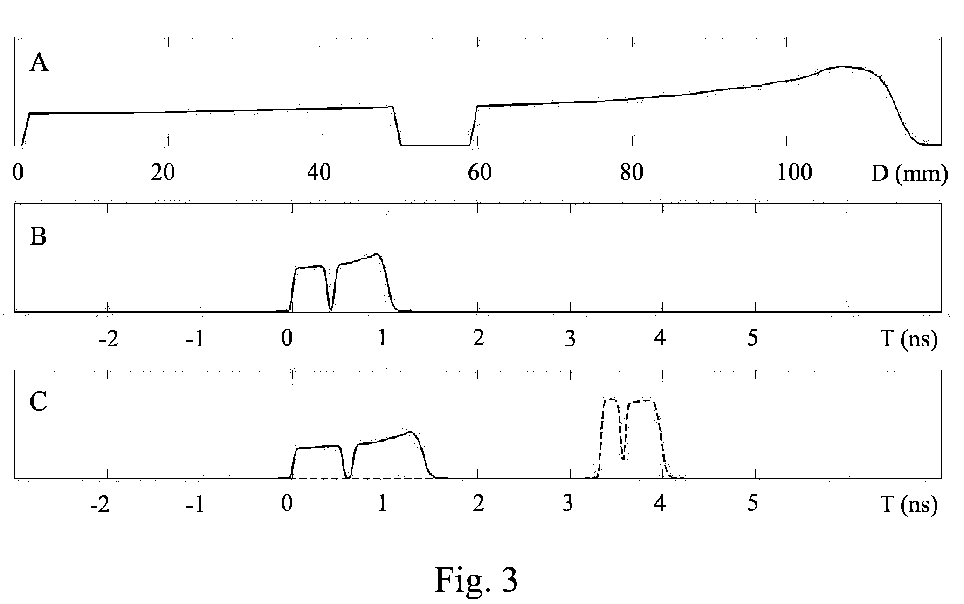

The inventors have investigated the robustness of the particle beam system according to the invention with respect to inhomogeneities in the target. As an example, the effect of an inhomogeneity is shown in Fig. 3 where an air gap with a thickness 10 mm is located at a depth in the target of 50 mm. In the top panel A, the number of prompt gammas produced as function of the penetration depth in the target are shown. At the location of the air gap, as shown, no prompt gammas are produced. This is then also observed in the middle panel B where the relative number of prompt gammas emitted as function of time after entering the target is plotted. The emission profile in the second panel B is showing a longer emission profile compared with the emission profile of Fig. 2. The third panel C shows that the air gap has also an effect on the shape of the first timing profile and the second timing profile.

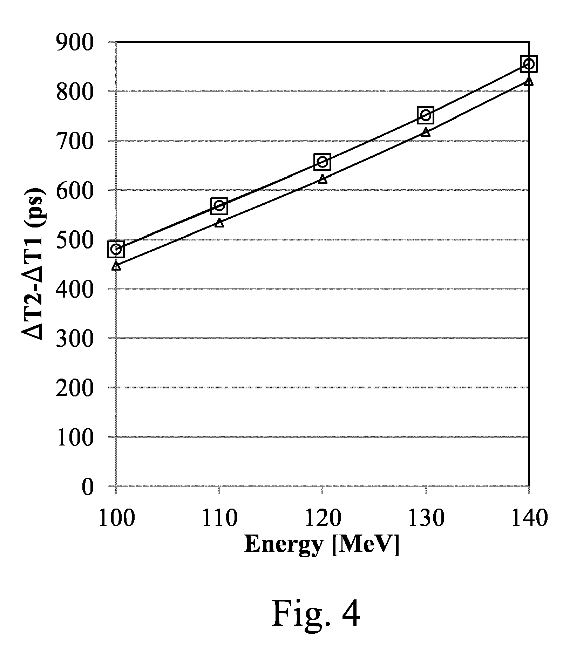

It has been found by the inventors that the location of inhomogeneities in the target have no effect on the accuracy for determining the particle beam depth. This is has been examined and is illustrated in Fig. 4 where $\triangle T2-\triangle T1$ is plotted for proton beams in a plastic target (more specifically a PMMA) having energies between 100 MeV and 140 MeV. Three cases have been evaluated and the results are plotted on the same Fig. 4: 1) circles correspond to a situation where there is a 5 mm cavity located at 1 cm depth in the target, 2) squares correspond to a situation where there is a 5 mm cavity located at 6 cm depth in the target and 3) triangles correspond to a situation where there is no cavity in the target. These results show that not only the 5 mm shift due to the cavity is clearly observed but also that there is no difference for what concerns the location of the cavity so that the particle depth determining method according to the invention shows to be robust.

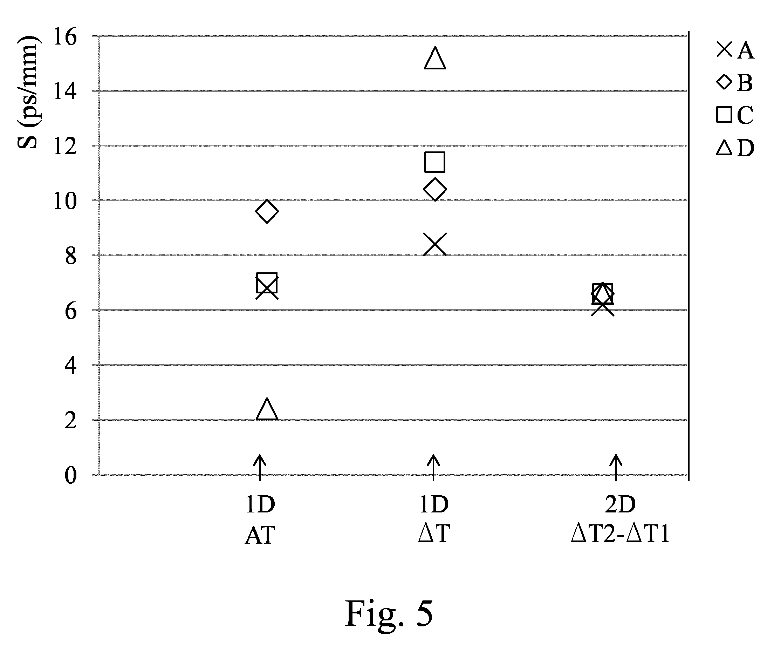

The robustness of the particle beam system and the method according to the invention has been further investigated by performing additional simulations. For the exemplary simulation, a beam of 140 MeV of protons was sent on a plastic target (PMMA). The beam was a bunched beam with a bunche duration of 40 ps (picoseconds). The expected particle penetration depth for a homogeneous target having an average density of 1,19 g/cm³ (=reference target)is 12,1 cm. To test the robustness of the method of range verification, four different modifications were made to the target to induce a same 5 mm (millimetre) shift of the penetration depth of the particle beam. The purpose of the test is to demonstrate that the system and method according to the invention can measure such a shift in penetration depth independent of the nature that causes the range shift. Those four modifications made to the target to induce a 5 mm shift in penetration depth, as indicated in Fig. 5, are the following: A) indicated with a cross, is related to target having an overall reduction of the density by 0,05 g/cm³ compared to the reference target, i.e. an average target density of 1,14 g/cm³ instead of the 1,19 g/cm³ for the reference target, B) indicated with a diamond, is related to a target having a cavity of 5mm located at a depth of 1 cm in the target, C) indicated with a square, is related to a target having a cavity of 5mm located at a depth of 6 cm in the target, D) indicated with a triangle, is related to a target having a cavity of 5mm located at a depth of 11 cm in the target. For the four cases A,B,C,D, it is expected that the proton penetration depth shifts with 5 mm compared to the reference target. The purpose of this test was to verify the sensitivity to detect such variations in penetration depth. The sensitivity is defined as the variation of a measured time signal per mm of change of penetration.

The results of the exemplary test simulations are shown in Fig. 5. For this exemplary simulation, a first detector was located at 180° and a second detector at 0° with respect to the beam direction. According to the invention, the values $\Delta T2-\Delta T1$ were determined for timing profiles obtained with the reference target and with the four modified targets (the cases A,B,C or D as discussed above). The sensitivity S of the detection method according to the invention is obtained by first calculating $(\Delta T2-\Delta T1)$ measured for the four modified targets (A,B,C or D) and subtracting the $(\Delta T2-\Delta T1)$ determined for the reference target and then in a second step dividing this difference in time by the expected penetration shift of 5 mm. This sensitivity S, expressed in ps (picoseconds) per mm (millimeter) is plotted in Fig.5 on the right side, at the coordinate position labeled "2D $\Delta T2-\Delta T1$". As shown in this example, the sensitivity using the method according to the invention is about 6,5 ps/mm. This means that one has to measure a time difference of 6,5 ps to detect a variation in penetration depth of one mm. What is important is that the same sensitivity is obtained for the four cases A,B,C,D, i.e. the sensitivity is independent of the nature of what caused the shift in penetration depth (e.g. an overall density change, a cavity in the target, ...).

To stress this important advantage of the invention where a particle penetration depth is verified independently of the nature causing a shift in penetration depth, an additional simulation is performed for a system and method that would only use one detector to measure the prompt gammas and hence only obtaining one prompt gamma timing profile. The results are also shown in Fig.5 where at the coordinate position labeled "1D $\Delta T$", the results of the sensitivity of a single detector are shown. In this case, the sensitivity is obtained by, in a first step, determining the difference between the width $\Delta T$ measured for a modified target (the case A,B,C or D discussed above) and subtracting the width $\Delta T$ determined for the reference target and then, in a second step, dividing this difference in time by the expected penetration shift of 5 mm. The width $\Delta T$ is defined and determined in the same way as was done for $\Delta T1$ and $\Delta T2$, discussed above (equation (1) or (2)). As shown in Fig. 5, for each of the four modified target cases A,B,C and D, four different sensitivity values S are obtained. In other words, when using only one detector for detecting the prompt gammas, the determination of penetration depth requires an exact knowledge of the target structure. For example, if there are cavities in the target one needs to know where they are located in the target.

The reason of this sensitivity variation observed when using only one detector and associated timing profile can be understood from equation (1) or (2), where it is shown that $\Delta T$ depends on Tbeam, the time the particle has to travel in the target. This particle travel time depends on the target structure. As the particle speed decreases when penetrating the target, the travel time will be different when for example a cavity is present in the distal part or the proximal part of the target. With the system and method according to the invention the penetration depth is deduced from a time difference $\Delta T2-\Delta T1$ independent from Tbeam.

Alternatively, when using only one detector, instead of determining the width $\Delta T$, one could also determine a average value "AT" of the timing profile. Similarly, the average value AT obtained for the reference target can then be compared with the value obtained for the modified targets (A,B,C,D as discussed above). The results of this approach are also shown in Fig 5 at the coordinate position labeled "1D AT". As shown, also with this approach the sensitivity for detecting a penetration depth variation is dependent on the target structure.

The timing setup and technology for measuring the two timing profiles with the first and the second detector can adopt technology that is currently used in standard time-of-flight (TOF) measurements used in for example particle beam physics or used for PET time-of-flight measurements (see e.g. Schaart et al. in Phys. Med. Biol. 55 (2010) N179-N189). The first gamma detector and the second gamma detector can be gamma detectors well known in the art such as for example detectors comprising a scintillation crystal and a photomultiplier readout.

With the particle beam system according to the invention, the timing profiles are preferably measured with a timing resolution equal or less than 10 ns (ten nanoseconds). In this way, when accounting for sufficient statistics in the timing profiles (for example, a few 100 to 1000 counts), time differences in the ps (picoseconds) time range can be measured. More preferably, the timing profiles are measured with a timing resolution equal or smaller than 1 ns (one nanoseconds).

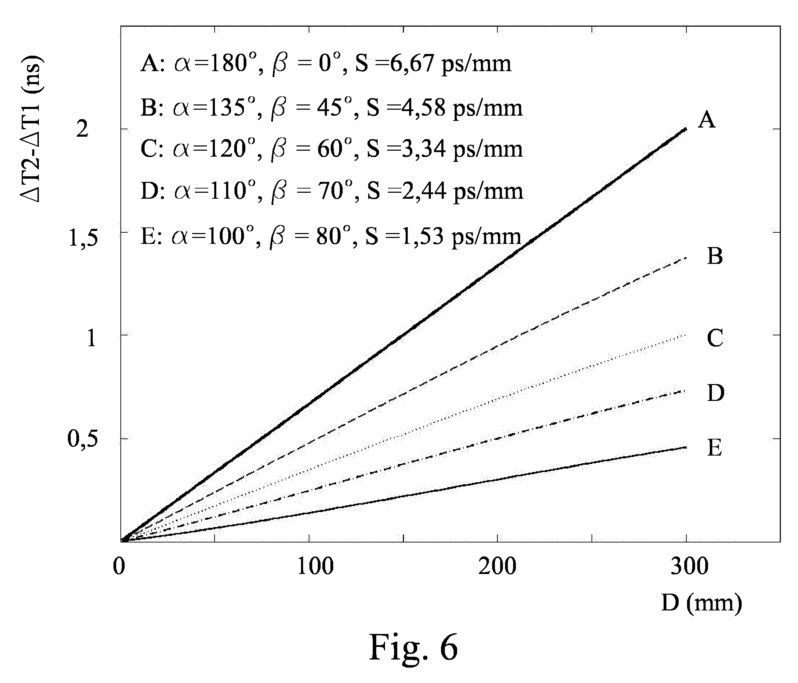

The relative geometry of the first and second detector is further discussed and illustrated in Fig. 6. According to the invention, the first and second detector are positioned at a different angle with respect to the beam direction. The method of the invention can be applied for any position of the two detectors, as long as the two detectors measure prompt gammas emitted from a different direction with respect to the beam direction. In Fig. 6, the time difference $\Delta T2-\Delta T1$ is plotted as function of the penetration depth D of the beam in the target for different detector setups labelled A,B,C,D,E. Each detector setup is defined by the angles $\alpha$ and $\beta$ defining the angle between the beam direction and the direction from the target to respectively the first and the second detector. The average sensitivity S for each of the configurations is also plotted on Fig.6. The highest sensitivity of 6,67 ps/mm is obtained for case A where the two detectors are separated by 180° (degree) and the lowest sensitivity of 1,53 ps/mm is obtained for case E where the two detectors are separated by an angle of 20°.

Preferably, the particle beam system according to the invention has the second gamma detector 5 located with respect to the first gamma detector 4 such that the first and the second detector are detecting prompt gammas emitted at angles that differ by at least 20°. The angles of emission of the prompt gammas are defined with respect to the beam direction.

More preferably, the first gamma detector is configured for detecting prompt gammas emitted at an angle equal or larger than 100° with respect to the beam direction and the second gamma detector is configured for detecting prompt gammas emitted at an angle equal or smaller than 80° with respect to the beam direction.

As already indicated above, according to a second aspect of the invention, a method is provided to determine a penetration depth of an energetic particle beam in a target by detecting prompt gammas produced when the energetic particle beam penetrates the target. As also discussed above, a particle beam is formed by particles or a bunch of particles.

As schematically illustrated in Fig. 1, a first gamma detector 4 and a second gamma detector 5 are provided for detecting prompt gammas emitted from the target. In the method according to the invention, the two prompt gamma detectors are located in a different location with respect to the target such that the first and the second detector are configured for detecting prompt gammas emitted at different angles with respect to the beam direction.

Further, a timing reference signal representing a time structure of the energetic particle beam is provided. According to the method of the invention, prompt gammas are measured with the first detector in synchrony with the timing reference signal so as to obtain a first timing profile. Similar, with the second detector, prompt gammas are measured with the second detector in synchrony with the timing reference signal so as to obtain a second timing profile.

According to the method of the invention, a first time width $\Delta T1$ is determined from the first timing profile and a second time width $\Delta T2$ is determined from the second timing profile. In a next step, a photon travel shift $\Delta DP$ is determined whereby the photon travel shift is defined as the distance travelled by a photon in the time interval equal to the difference between the first and the second time width:

$$\Delta DP = (\Delta T2 - \Delta T1) * c$$

whereby c is equal to the speed of light.

Finally, according to the method of the invention, the penetration depth is determined by correlating the photon travel shift $\Delta DP$ with the difference in detector location between the first and second detector.

Preferably, the method of the invention comprises a calculating step for calculating a distance R in the target by solving the equation

$$\Delta DP = \sqrt{(R * R + d1 * d1 - 2 * R * d1 * \cos(\alpha)} - \sqrt{(R * R + d2 * d2 - 2 * R * d2 * \cos(\beta)} - (d1 - d2)$$

whereby

$\Delta DP$ is the said photon travel shift

d1 is the distance from the first detector to the entrance point, the entrance point being defined as the point where the energetic particle beam enters the target;

d2 is the distance from the second detector to the entrance point;

$\alpha$ is the angle between the beam direction and the direction going from the entrance point towards the first detector;

$\beta$ is the angle between the beam direction and the direction going from the entrance point towards the second detector.

Preferably, in the method according to the invention, the second gamma detector (5) is located with respect to the first detector (4) such that the first and the second detector are detecting prompt gammas emitted at angles with respect to the beam direction (6) that differ by at least 20°.

More preferably, in the method according to the invention, the first gamma detector (4) is located for measuring prompt gammas emitted at an angle equal or larger than 100° with respect to the beam direction and the second gamma detector (5) is located for measuring prompt gammas emitted at an angle equal or smaller than 80° with respect to the beam direction.

The present invention has been described in terms of specific embodiments, which are illustrative of the invention and not to be construed as limiting. More generally, it will be appreciated by persons skilled in the art that the present invention is not limited by what has been particularly shown and/or described hereinabove. The invention resides in each and every novel characteristic feature and each and every combination of characteristic features. Reference numerals in the claims do not limit their protective scope.

**Claims**

1. A particle beam system for delivering energetic particles or bunches of energetic particles to a target comprising

   • a particle beam source (7) for delivering energetic particles or bunches of energetic particles;
   • beam directing means (8) for directing the energetic particles or bunches of energetic particles in a beam direction (6) pointing to said target (2);
   • a timing controller (3) configured for providing a timing reference signal representing a time structure for delivering said energetic particles or bunches of energetic particles;
   • a first gamma detector (4) configured for detecting prompt gammas emitted from said target;
   • a second gamma detector (5) configured for detecting prompt gammas emitted from said target and located with respect to said first detector such that the first and the second detector are detecting prompt gammas emitted from different angles with respect to said beam direction (6);
   • a data acquisition system (9) for measuring prompt gammas in synchrony with the said timing reference signal so as to obtain a timing profile indicating a number of prompt gammas measured as a function of a time elapsed since the delivery of a particle or a bunch of particles, said first and second detector are coupled to said data acquisition system so as to acquire a first timing profile from the first detector and to acquire a second timing profile from the second detector;

   **characterized in that** said particle beam system further comprises

   • a data analyser (10) comprising an algorithm configured for

     ◦ determining from said first timing profile a first time width $\Delta$T1;
     ◦ determining from said second timing profile a second time width $\Delta$T2;
     ◦ determining a photon travel shift $\Delta$DP defined as a distance travelled by a photon in the time interval equal to the difference between the first and the second time width:

$$\Delta DP \; = \; (\Delta T2 - \Delta T1) * c$$

   whereby c is equal to the speed of light;
     ◦ determining a particle beam penetration depth by correlating said photon travel shift $\Delta$DP with the difference in detector location between the first and second detector.

2. A particle beam system according to claim 1 wherein said data analyser (10) comprises computing means configured for calculating a distance R in the target by solving the equation

$$\Delta DP \; = \; \sqrt{(R * R + d1 * d1 - 2 * R * d1 * \cos(\alpha)} \; - $$
$$\sqrt{(R * R + d2 * d2 - 2 * R * d2 * \cos(\beta)} \; - (d1 - d2)$$

   whereby

   $\Delta$DP is the said photon travel shift;
   d1 is the distance from the first detector to the entrance point, the entrance point being defined as the point where the energetic particles or bunches of energetic particles enter the target;
   d2 is the distance from the second detector to the entrance point;
   $\alpha$ is the angle between the beam direction and the direction going from the entrance point towards the first detector;
   $\beta$ is the angle between the beam direction and the direction going from the entrance point towards the second detector.

3. A particle beam system according to any of previous claims wherein said second gamma detector (5) is located with respect to said first gamma detector (4) such that the first and the second gamma detector are detecting prompt gammas emitted at angles with respect to the beam direction (6) that differ by at least 20°.

4. A particle beam system according to any of previous claims wherein said first gamma detector is located for measuring prompt gammas emitted at an angle equal or larger than 100° with respect to the beam direction and said second gamma detector is located for measuring prompt gammas emitted at an angle equal or smaller than 80° with respect to the beam direction.

5. A particle beam system according to any of previous claims wherein said data acquisition system (9) is configured to measure said timing profiles with a resolution equal or smaller than ten nanoseconds.

6. A particle beam system according to any of the previous claims wherein said particle beam source is comprising a particle accelerator for delivering protons or ions having an energy larger than 40 MeV per mass unit.

7. A method for verifying a penetration depth of an energetic particle beam (1) in a target (2) by detecting prompt gammas produced when said energetic particle beam penetrates said target, the method comprising the steps of

   • providing a first gamma detector (4) configured for detecting prompt gammas;
   • providing a second gamma detector (5) configured for detecting prompt gammas;
   • locating said first and second detector in a different location with respect to the target such that the first and the second detector are configured for detecting prompt gammas emitted at different angles with respect a direction of the energetic particle beam;
   • providing a timing reference signal representing a time structure of the energetic particle beam;
   • measuring prompt gammas emitted from the target with said first detector in synchrony with said timing reference signal so as to obtain a first timing profile;
   • measuring prompt gammas emitted from the target with said second detector in synchrony with said timing reference signal so as to obtain a second timing profile;

   **characterized in that** the method further comprises the steps of

   • determining from said first timing profile a first time width $\Delta T1$;
   • determining from said second timing profile a second time width $\Delta T2$;
   • determining a photon travel shift $\Delta DP$ defined as a distance travelled by a photon in the time interval equal to the difference between the first and the second time width:

$$\Delta DP = (\Delta T2 - \Delta T1) * c$$

   whereby c is equal to the speed of light;
   • determining said penetration depth by correlating said photon travel shift $\Delta DP$ with the difference in detector location between the first and second detector.

8. A method according to claim 7 whereby the step of determining said penetration depth comprises a step of calculating a distance R in the target by solving the equation

$$\Delta DP = \sqrt{(R * R + d1 * d1 - 2 * R * d1 * \cos(\alpha)} - \\ \sqrt{(R * R + d2 * d2 - 2 * R * d2 * \cos(\beta)} - (d1 - d2)$$

   whereby

   $\Delta DP$ is the said photon travel shift
   d1 is the distance from the first detector to the entrance point, the entrance point being defined as the point where the energetic particle beam enters the target;
   d2 is the distance from the second detector to the entrance point;
   $\alpha$ is the angle between the beam direction and the direction going from the entrance point towards the first detector;
   $\beta$ is the angle between the beam direction and the direction going from the entrance point towards the second detector.

9. A method according to any of claims 7 or 8 wherein said second gamma detector (5) is located with respect to said first detector (4) such that the first and the second detector are detecting prompt gammas emitted at angles with respect to the beam direction (6) that differ by at least 20°.

10. A method according to any of claims 7 or 8 wherein said first gamma detector (4) is located for measuring prompt gammas emitted at an angle equal or larger than 100° with respect to the beam direction and said second gamma detector (5) is located for measuring prompt gammas emitted at an angle equal or smaller than 80° with respect to the beam direction.

**Patentansprüche**

1. Teilchenstahlsystem zur Lieferung von energetischen Teilchen oder von Bündeln energetischer Teilchen an ein Ziel, umfassend:

   • eine Teilchenstrahlquelle (7) zur Lieferung von energetischen Teilchen oder von Bündeln energetischer Teilchen;
   • eine Strahllenkungseinrichtung (8) zur Lenkung der energetischen Teilchen oder der Bündel energetischer Teilchen in eine Strahlrichtung (6), die in Richtung des Ziels (2) zeigt;
   • eine Zeitsteuerungsvorrichtung (3), die konfiguriert ist, um ein Zeitreferenzsignal bereitzustellen, das eine Zeitstruktur zur Lieferung der energetischen Teilchen oder der Bündel energetischer Teilchen darstellt;
   • einen ersten Gamma-Detektor (4), der konfiguriert ist, um von dem Ziel emittierte prompte Gammastrahlen festzustellen;
   • einen zweiten Gamma-Detektor (5), der konfiguriert ist, um von dem Ziel emittierte prompte Gammastrahlen festzustellen, und der bezüglich des ersten Detektors derart positioniert ist, sodass der erste und der zweite Detektor prompte Gammastrahlen feststellen, die bezüglich der Strahlrichtung (6) aus unterschiedlichen Winkeln emittiert werden;
   • ein Datenerfassungssystem (9) zur Messung prompter Gammastrahlen synchron zu dem Zeitreferenzsignal, um ein Zeitprofil zu erhalten, das eine Vielzahl von prompten gemessenen Gammastrahlen als eine Funktion einer verstrichenen Zeit seit der Lieferung eines Teilchens oder eines Bündels von Teilchen angibt, wobei der erste und der zweite Detektor mit dem Datenerfassungssystem gekoppelt sind, um ein erstes Zeitprofil von dem ersten Detektor und ein zweites Zeitprofil von dem zweiten Detektor zu erhalten;

   **dadurch gekennzeichnet, dass** das Teilchenstrahlsystem ferner Folgendes umfasst:

   • eine Datenanalysevorrichtung (10), die einen Algorithmus umfasst, der konfiguriert ist, um

      ○ aus dem ersten Zeitprofil eine erste Zeitbreite ΔT1 zu bestimmen;
      ○ aus dem zweiten Zeitprofil eine zweite Zeitbreite ΔT2 zu bestimmen;
      ○ eine Verschiebung der Photonenbewegung ΔDP zu bestimmen, die als eine Distanz beschrieben wird, die ein Photon in dem Zeitintervall zurücklegt, das der Differenz aus der zweiten und der ersten Zeitbreite entspricht:

$$\Delta DP = (\Delta T2 - \Delta T1) * c$$

   wobei c der Lichtgeschwindigkeit entspricht;
      ○ eine Teilchenstrahl-Durchdringungstiefe durch Korrelation der Verschiebung der Photonenbewegung ΔDP mit der Differenz aus den Positionen der Detektoren zwischen dem ersten und dem zweiten Detektor zu bestimmen.

2. Teilchenstrahlsystem nach Anspruch 1, wobei die Datenanalysevorrichtung (10) Rechenmittel umfasst, die konfiguriert sind, um eine Distanz R in dem Ziel durch das Lösen der folgenden Gleichung zu berechnen:

$$\Delta DP = \sqrt{(R * R + d1 * d1 - 2 * R * d1 * \cos(\alpha)} -$$

$$\sqrt{(R * R + d2 * d2 - 2 * R * d2 * \cos(\beta)} - (d1-d2)$$

wobei

$\Delta$DP die Verschiebung der Photonenbewegung ist;

d1 die Distanz von dem ersten Detektor zu dem Eintrittspunkt ist, wobei der Eintrittspunkt als der Punkt definiert ist, an dem die energetischen Teilchen oder die Bündel energetischer Teilchen in das Ziel eintreten;

d2 die Distanz von dem zweiten Detektor zu dem Eintrittspunkt ist;

$\alpha$ der Winkel zwischen der Strahlrichtung und der Richtung ist, die von dem Eintrittspunkt in Richtung des ersten Detektors verläuft;

$\beta$ der Winkel zwischen der Strahlrichtung und der Richtung ist, die von dem Eintrittspunkt in Richtung des zweiten Detektors verläuft.

3. Teilchenstrahlsystem nach einem der vorhergehenden Ansprüche, wobei der zweite Gamma-Detektor (5) bezüglich des ersten Gamma-Detektors (4) derart positioniert ist, sodass der erste und der zweite Gamma-Detektor prompte Gammastrahlen feststellen, die in Winkeln bezüglich der Strahlrichtung (6) emittiert werden, die sich um mindestens 20° unterscheiden.

4. Teilchenstrahlsystem nach einem der vorhergehenden Ansprüche, wobei der erste Gamma-Detektor zur Messung prompter Gammastrahlen positioniert ist, die bezüglich der Strahlrichtung in einem Winkel von 100° oder mehr emittiert werden, und der zweite Gamma-Detektor zur Messung prompter Gammastrahlen positioniert ist, die bezüglich der Strahlrichtung in einem Winkel von 80° oder weniger emittiert werden.

5. Teilchenstrahlsystem nach einem der vorhergehenden Ansprüche, wobei das Datenerfassungssystem (9) konfiguriert ist, um die Zeitprofile mit einer Auflösung von zehn Nanosekunden oder weniger zu messen.

6. Teilchenstrahlsystem nach einem der vorhergehenden Ansprüche, wobei die Teilchenstrahlquelle einen Teilchenbeschleuniger zur Lieferung von Protonen oder Ionen umfasst, die eine Energie von über 40 MeV pro Masseeinheit aufweisen.

7. Verfahren zur Verifizierung einer Durchdringungstiefe eines energetischen Teilchenstrahls (1) in einem Ziel (2) durch Feststellen prompter Gammastrahlen, die erzeugt werden, wenn der energetische Teilchenstrahl das Ziel durchdringt, wobei das Verfahren die folgenden Schritte umfasst:

• Bereitstellen eines ersten Gamma-Detektors (4), der konfiguriert ist, um prompte Gammastrahlen festzustellen;

• Bereitstellen eines zweiten Gamma-Detektors (5), der konfiguriert ist, um prompte Gammastrahlen festzustellen;

• Positionieren des ersten und des zweiten Detektors an einer bezüglich des Ziels unterschiedlichen Position, sodass der erste und der zweite Detektor konfiguriert sind, um prompte Gammastrahlen festzustellen, die bezüglich einer Richtung des energetischen Teilchenstrahls in unterschiedlichen Winkeln emittiert werden;

• Bereitstellen eines Zeitreferenzsignals, das eine Zeitstruktur des energetischen Teilchenstrahls darstellt;

• Messen prompter Gammastrahlen, die von dem Ziel emittiert werden, mit dem ersten Detektor synchron zu dem Zeitreferenzsignal, um ein erstes Zeitprofil zu erhalten;

• Messen prompter Gammastrahlen, die von dem Ziel emittiert werden, mit dem zweiten Detektor synchron zu dem Zeitreferenzsignal, um ein zweites Zeitprofil zu erhalten;

**dadurch gekennzeichnet, dass** das Verfahren ferner folgende Schritte umfasst:

• Bestimmen einer ersten Zeitbreite $\Delta$T1 aus dem ersten Zeitprofil;

• Bestimmen einer zweiten Zeitbreite $\Delta$T2 aus dem zweiten Zeitprofil;

• Bestimmen einer Verschiebung der Photonenbewegung $\Delta$DP, die als eine Distanz beschrieben wird, die ein Photon in dem Zeitintervall zurücklegt, das der Differenz aus der zweiten und der ersten Zeitbreite entspricht:

$$\Delta DP = (\Delta T2 - \Delta T1) * c$$

wobei c der Lichtgeschwindigkeit entspricht;
• Bestimmen der Teilchenstrahl-Durchdringungstiefe durch Korrelation der Verschiebung der Photonenbewegung ΔDP mit der Differenz aus den Positionen der Detektoren zwischen dem ersten und dem zweiten Detektor.

8. Verfahren nach Anspruch 7, wobei der Schritt des Bestimmens der Durchdringungstiefe einen Schritt des Berechnens einer Distanz R in dem Ziel durch Lösen der folgenden Gleichung umfasst:

$$\Delta DP = \sqrt{(R * R + d1 * d1 - 2 * R * d1 * \cos(\alpha)} -$$
$$\sqrt{(R * R + d2 * d2 - 2 * R * d2 * \cos(\beta)} - (d1-d2)$$

wobei

ΔDP die Verschiebung der Photonenbewegung ist;
d1 die Distanz von dem ersten Detektor zu dem Eintrittspunkt ist, wobei der Eintrittspunkt als der Punkt definiert ist, an dem der energetische Teilchenstrahl in das Ziel eintritt;
d2 die Distanz von dem zweiten Detektor zu dem Eintrittspunkt ist;
α der Winkel zwischen der Strahlrichtung und der Richtung ist, die von dem Eintrittspunkt in Richtung des ersten Detektors verläuft;
β der Winkel zwischen der Strahlrichtung und der Richtung ist, die von dem Eintrittspunkt in Richtung des zweiten Detektors verläuft.

9. Verfahren nach einem der Ansprüche 7 oder 8, wobei der zweite Gamma-Detektor (5) bezüglich des ersten Gamma-Detektors (4) derart positioniert ist, sodass der erste und der zweite Gamma-Detektor prompte Gammastrahlen feststellen, die in Winkeln bezüglich der Strahlrichtung (6) emittiert werden, die sich um mindestens 20° unterscheiden.

10. Verfahren nach einem der Ansprüche 7 oder 8, wobei der erste Gamma-Detektor (4) zur Messung prompter Gammastrahlen positioniert ist, die bezüglich der Strahlrichtung in einem Winkel von 100° oder mehr emittiert werden, und der zweite Gamma-Detektor (5) zur Messung prompter Gammastrahlen positioniert ist, die bezüglich der Strahlrichtung in einem Winkel von 80° oder weniger emittiert werden.

**Revendications**

1. Système de faisceau de particules, destiné à délivrer des particules énergétiques ou des paquets de particules énergétiques à une cible, comprenant

• une source de faisceau de particules (7) destiné à délivrer des particules énergétiques ou des paquets de particules énergétiques ;
• des moyens de direction de faisceau (8) destiné à diriger les particules énergétiques ou les paquets de particules énergétiques dans une direction de faisceau (6) pointant vers ladite cible (2) ;
• un dispositif de commande de synchronisation (3) configuré pour produire un signal de référence de synchronisation représentant une structure temporelle destinée à délivrer lesdites particules énergétique ou paquets de particules énergétiques ;
• un premier détecteur de rayons gamma (4) configuré pour détecter des rayons gamma instantanés émis par ladite cible ;
• un second détecteur de rayons gamma (5) configuré pour détecter des rayons gamma instantanés émis par ladite cible et placé par rapport audit premier détecteur de telle sorte que les premier et second détecteurs détectent les rayons gammas instantanés émis sous des angles différents par rapport à ladite direction de faisceau (6) ;
• un système d'acquisition de données (9) destiné à mesurer les rayons gammas instantanés en synchronisation avec ledit signal de référence de synchronisation de manière à obtenir un profil de synchronisation indiquant un nombre de rayons gammas instantanés mesurés en fonction du temps écoulé depuis la délivrance d'une particule ou d'un paquet de particules, lesdites premier et second détecteurs sont couplés audit système d'acquisition de données afin d'acquérir un premier profil de synchronisation provenant du premier détecteur et d'acquérir un second profil de synchronisation provenant du second détecteur ;

**Caractérisé en ce que** ledit système de faisceau de particules comprend en outre

• un analyseur de données (10) comprenant un algorithme configuré pour

◦ déterminer à partir dudit premier profil de synchronisation une première largeur temporelle ∆T1 ;
◦ déterminer à partir dudit second profil de synchronisation une largeur temporelle ∆T2 ;
◦ déterminer un décalage de déplacement photon ∆DP défini comme étant la distance parcourue par un photon dans l'intervalle de temps égal à la différence entre les première et seconde largeurs temporelle :

$$\Delta DP = (\Delta T2 - \Delta T1) * c$$

où c est égal à la vitesse de la lumière ;
◦ déterminer une profondeur de pénétration de faisceau de particules par corrélation dudit décalage de déplacement de photon ∆DP à la différence d'emplacement de détecteur entre les premier et second détecteurs.

2. Système de faisceau de particules selon la revendication 1, dans lequel ledit analyseur de données (10) comprend des moyens de calcul configuré pour calculer une distance R dans la cible par résolution de l'équation

$$\Delta DP = \sqrt{(R * R + d1 - 2 * R * d1 * \cos(\alpha)} - \sqrt{(R * R + d2 - 2 * R * d2 * \cos(\alpha)} - (d1 - d2)$$

où

∆DP est ledit décalage de déplacement de photon ;
d1 est la distance entre le premier détecteur et le point d'entrée, le point d'entrée étant défini comme étant le point où les particules énergétiques ou les paquets de particules énergétiques entrent dans la cible ;
d2 est la distance entre le second détecteur et le point d'entrée ;
α est l'angle entre la direction de faisceau et la direction allant du point d'entrée au premier détecteur ;
β est l'angle entre la direction de faisceau et la direction allant du point d'entrée au second détecteur.

3. Système de faisceau de particules selon l'une quelconque des revendications précédentes, dans lequel ledit second détecteur de rayons gamma (5) est disposé par rapport audit premier détecteur de rayons gamma (4) de telle sorte que les premier et second détecteurs de rayons gamma détectent des rayons gamma instantanés émis à des angles par rapport à la direction de faisceau (6) qui diffèrent d'au moins 20°.

4. Système de faisceau de particules selon l'une quelconque des revendications précédentes, dans lequel ledit premier détecteur de rayons gamma est placé de manière à mesurer des rayons gamma instantanés émis avec un angle égal ou supérieur à 100° par rapport à la direction de faisceau et ledit second détecteur de rayons gamma est placé de manière à mesurer les rayons gammas instantanés émis avec un angle égal ou inférieur à 80° par rapport à la direction de faisceau.

5. Système de faisceau de particules selon l'une quelconque des revendications précédentes, dans lequel ledit système d'acquisition de données (9) est configuré pour mesurer ledit profil de synchronisation avec une résolution égale ou inférieure à dix nanosecondes.

6. Système de faisceau de particules selon l'une quelconque des revendications précédentes, dans lequel ladite source de faisceau de particules comprend un accélérateur de particules destiné à délivrer des protons ou des ions ayant une énergie supérieure à 40 MeV par unité de masse.

**7.** Procédé de vérification de la profondeur de pénétration d'un faisceau de particules énergétiques (1) dans une cible (2) par détection de rayons gammas instantanés produits lorsque ledit faisceau de particules énergétiques pénètre dans ladite cible, le procédé comprenant les étapes consistant à

- produire un premier détecteur de rayons gamma (4) configuré pour détecter des rayons gammas instantanés ;
- produire un second détecteur de rayons gamma (5) configuré pour détecter des rayons gammas instantanés ;
- positionner lesdits premier et second détecteurs à un emplacement différent par rapport à la cible de telle sorte que les premier et second détecteurs sont configurés pour détecter des rayons gamma instantanés émis à des angles différents par rapport à la direction du faisceau de particules énergétiques ;
- produire un signal de référence de synchronisation représentant une structure temporelle du faisceau de particules énergétiques ;
- mesurer les rayons gamma instantanés émis par la cible avec ledit premier détecteur en synchronisation avec ledit signal de référence de synchronisation de manière à obtenir un premier profil de synchronisation ;
- mesurer les rayons gamma instantanés émis par la cible avec ledit second détecteur en synchronisation avec ledit signal de référence de synchronisation de manière à obtenir un second profil de synchronisation ; **caractérisé en ce que** le procédé comprend en outre les étapes consistant à
- déterminer à partir dudit premier profil de synchronisation une première largeur temporelle $\Delta T1$ ;
- déterminer à partir dudit second profil de synchronisation une seconde largeur temporelle $\Delta T2$ ;
- déterminer un décalage de déplacement de photon $\Delta DP$ défini comme étant la distance parcourue par un photon dans l'intervalle de temps égal à la différence entre les première et seconde largeurs temporelle :

$$\Delta DP = (\Delta T2 - \Delta T1) * c$$

où c est égal à la vitesse de la lumière ;
- déterminer ladite profondeur de pénétration par corrélation dudit décalage de déplacement de photon $\Delta DP$ avec la différence d'emplacement de détecteurs entre les premier et second détecteurs.

**8.** Procédé selon la revendication 7, dans lequel l'étape consistant à déterminer ladite profondeur de pénétration comprend une étape consistant à calculer une distance R dans la cible par résolution de l'équation

$$\Delta DP = \sqrt{(R * R + d1 * d1 - 2 * R * d1 * \cos(\alpha)} - \sqrt{(R * R + d2 - 2 * R * d2 * \cos(\alpha)}$$

$$- (d1 - d2)$$

où

$\Delta DP$ est ledit décalage de déplacement de photon ;
d1 est la distance entre le premier détecteur et le point d'entrée, le point d'entrée étant défini comme étant le point où le faisceau de particules énergétiques entre dans la cible ;
d2 est la distance entre le second détecteur et le point d'entrée ;
$\alpha$ est l'angle entre la direction de faisceau et la direction allant du point d'entrée au premier détecteur ;
$\beta$ est l'angle entre la direction de faisceau et la direction allant du point d'entrée au second détecteur.

**9.** Procédé selon l'une quelconque des revendications 7 ou 8, dans lequel ledit second détecteur de rayons gamma (5) est placé par rapport audit premier détecteur (4) de telle sorte que les premier et second détecteurs détectent les rayons gammas instantanés émis à des angles par rapport à la direction de faisceau (6) qui diffèrent d'au moins 20°.

**10.** Procédé selon l'une quelconque des revendications 7 ou 8, dans lequel ledit premier détecteur de rayons gamma (4) est placé de manière à mesurer les rayons gammas instantanés émis à un angle égal ou supérieur à 100° par rapport à la direction de faisceau et ledit second détecteur de rayons gamma (5) est placé de manière à mesurer les rayons gammas instantanés émis à un angle égal ou inférieur à 80° par rapport à la direction de faisceau.

Fig. 1

EP 2 950 119 B1

Fig. 2

Fig. 3

EP 2 950 119 B1

Fig. 4

EP 2 950 119 B1

Fig. 5

EP 2 950 119 B1

A: $\alpha = 180^\circ$, $\beta = 0^\circ$, S = 6,67 ps/mm

B: $\alpha = 135^\circ$, $\beta = 45^\circ$, S = 4,58 ps/mm

C: $\alpha = 120^\circ$, $\beta = 60^\circ$, S = 3,34 ps/mm

D: $\alpha = 110^\circ$, $\beta = 70^\circ$, S = 2,44 ps/mm

E: $\alpha = 100^\circ$, $\beta = 80^\circ$, S = 1,53 ps/mm

Fig. 6

EP 2 950 119 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8481951 B2
- WO 2012104416 A1
- WO 2012152938 A2
- US 2012025076 A1

**Non-patent literature cited in the description**

- **GUETH et al.** *Phys. Med. Biol.,* 2013, vol. 58, 4563-4577
- **SCHAART et al.** *Phys. Med. Biol.,* 2010, vol. 55, N179-N189